# EUROPÄISCHE PATENTSCHRIFT

(11) **EP 0 828 691 B1**
(45) Veröffentlichungstag und Bekanntmachung des Hinweises auf die Patenterteilung: **20.08.2003**
(21) Anmeldenummer: 96916061.3
(22) Anmeldetag: 14.05.1996
(51) Int. Cl.: C01G 51/06, C01G 51/04, C25B 1/00

(54) **VERFAHREN ZUR HERSTELLUNG VON BASISCHEN KOBALT(II)CARBONATEN**
PROCESS FOR PREPARING BASIC COBALTOUS CARBONATES
PROCEDE DE PREPARATION DE CARBONATES COBALTEUX BASIQUES

(30) Priorität: 26.05.1995 DE 19519328
(43) Veröffentlichungstag der Anmeldung: 18.03.1998
(73) Patentinhaber: H.C. Starck GmbH, 38642 Goslar (DE)
(72) Erfinder: GÖRGE, Astrid, D-38640 Goslar (DE); MEESE-MARKTSCHEFFEL, Juliane, D-38642 Goslar (DE); NAUMANN, Dirk, D-38667 Bad Harzburg (DE); OLBRICH, Armin, D-38723 Seesen (DE); SCHRUMPF, Frank, D-38640 Goslar (DE)
(74) Vertreter: Zobel, Manfred, Dr.
(86) Internationale Anmeldenummer: EP9602051
(87) Internationale Veröffentlichungsnummer: WO96037437

(56) Entgegenhaltungen:
- DATABASE WPI Derwent Publications Ltd., London, GB; AN 76-41538x XP002011990 & SU,A,476 232 (RYAZANOV) , 3.Dezember 1975
- DATABASE WPI Derwent Publications Ltd., London, GB; AN 80-32398c XP002011991 & SU,A,684 003 (SHCHERBINA) , 9.September 1979
- Gmelins Handbuch der Anorganischen Chemie, 8e Auflage, Kobalt, Teil A, 1932, Verlag Chemie, Berlin, Seiten 237,238,347,349

## Beschreibung

Die vorliegende Erfindung betrifft Verfahren zur Herstellung basischer Kobalt(II)-carbonate der allgemeinen Formel Co[(OH)₂]ₐ[CO₃]₁₋ₐ.

Für eine Reihe industrieller Anwendungen wird phasenreines Kobalt(II)hydroxid benötigt. So kann es zum Beispiel direkt oder nach vorangegangener Kalzination zu Kobalt(II)oxid als Komponente in der positiven Elektrode von modernen Hochleistungssekundärbatterien auf Basis Nickel/Cadmium oder Nickel/Metallhydrid eingesetzt werden.

Über intermediär entstehende Kobaltate(II), die im alkalischen Elektrolyten der Batterie (30 Gew.-% KOH) löslich sind, wird es gleichmäßig in der Elektrodenmasse verteilt und dort durch Oxidation in den sogenannten Formierzyklen als elektrisch leitfähige CoO(OH)-Schicht auf den Nickelhydroxidpartikeln abgeschieden. Im Ausgangsmaterial vorhandene Kobalt(III)-Anteile bilden keine löslichen Kobaltate und sind daher nicht nutzbar.

Der Einsatz von Kobaltverbindungen in alkalischen Sekundärbatterien auf Basis Nickel/Cadmium oder Nickel/Metallhydrid ist in der EP-A 353837 offenbart. Reine Kobalt(II)oxide werden auch als Katalysator und in der Elektronik eingesetzt.

Für die Herstellung von Kobalt(II)salzen schwacher Säuren werden entprechend reine basische Kobalt(II)carbonate oder -hydroxide eingesetzt.

Kobalt(II)hydroxid läßt sich durch Ausfällen aus wäßrigen Kobalt(II)salzlösungen mit Alkalilaugen herstellen. Die entstehenden Niederschläge besitzen in der Regel eine gelartige Konsistenz und sind schlecht filtrierbar und somit schwer neutralsalzfrei zu waschen. Außerdem sind sie in alkalischen Medien sehr oxidationsempfindlich, so daß die Filtrations- und Waschprozesse unter sorgfältigem Ausschluß von Luftsauerstoff durchgeführt werden müssen.

Weniger oxidationsempfindlich sind basische Kobalt(ll)carbonate. Diese können durch Fällung aus Kobalt(II)salzlösungen mit Alkali- und/oder Ammoniumcarbonatlösungen hergestellt werden. Bei der Fällung entstehen zwangsläufig äquimolare Mengen Neutralsalze. Um die gewonnenen basischen Kobalt(II)carbonate weitgehend neutralsalzfrei zu waschen, müssen große Waschwassermengen von bis zu 100 I pro kg Kobalt eingesetzt werden.

Für die Herstellung von hochkonzentrierten Kobalt(ll)salzlösungen mit Gehalten von 100 bis 200 g Co/l, wie sie für die beschriebenen Fällungen eingesetzt werden, kommen in der Regel nur unreine Kobaltrohstoffe in Frage, wie sie zum Beispiel bei der Aufarbeitung kobalthaltiger Schrotte anfallen. Der vergleichsweise niedrige Preis des Kobalts in diesen Schrotten wird durch die aufwendigen Reinzigungsprozesse zum Teil wieder aufgezehrt.

Hochreine Kobaltrohstoffe, wie sie zum Beispiel in Form von Kathoden umweltfreundlich und wirtschaftlich durch elektrolytische Reinigung erhältlich sind, lösen sich selbst in hochkonzentrierten, heißen Mineralsäuren nur mit unbefriedigenden Raum-Zeit-Ausbeuten.

Zur neutralsalzarmen Herstellung von Kobalthydroxiden bietet sich die anodische Oxidation in einer Elektrolyse an. Durch die Kreisführung der die Neutralsalze enthaltenden Elektrolytlösung wird die Abgabe dieser Salze an die Umwelt minimiert.

Derartige Elektrolysen sind zum Beispiel in Gmelins Handbuch der Anorganischen Chemie, 8. Auflage (1932), Kobalt, Teil A, Seiten 237, 238, 347, 349, sowie Ergänzungsband (1961), Seiten 314 bis 319, beschrieben. Auf diese Weise erzeugtes Kobalt(II)hydroxid wird sehr leicht schon in der Elektrolysezelle zum Kobalt(III)-hydroxid bzw. Kobalt(III)oxidhydroxid CoO(OH) oxidiert. Außerdem sind diese Niederschläge schwer filtrierbar und die Neutralsalzverunreinigungen im Produkt lassen sich nur durch hohen Waschwassereinsatz senken. Die so erreichbaren Reinheiten bleiben in der Regel dennoch unbefriedigend.

Aufgabe der vorliegenden Erfindung war es daher, ein Verfahren zur Herstellung basischer Kobalt(II)carbonate oder Kobalt(II)oxalatcarbonate bereitzustellen, welches die beschriebenen Nachteile des Standes der Technik, besonders in ökologischer Hinsicht, nicht aufweist.

Es wurde gefunden, daß die Oxidation des Kobalts zu Kobalt(III) während der elektrolytischen Umsetzung unterbunden wird, wenn der pH-Wert der Elektrolytlösung durch Pufferung mit dem System CO₃²⁻/HCO₃⁻/CO₂ im schwach sauren bis schwach alkalischen Bereich stabilisiert wird. Durch das Angebot an Hydrogencarbonat- und Carbonatanionen neben Hydroxidanionen in der Elektrolytlösung bildet das anodisch oxidierte Kobalt die im Vergleich zum Kobalt(II)hydroxid oxidationsstabileren, basischen Carbonate der allgemeinen Formel

Co[(OH)₂]ₐ[CO₃]₁₋ₐ

oder basische Oxalatcarbonate der allgemeinen Formel

Co[(OH)₂]ₐ[C₂O₄]_{b}[CO₃]_{1-a-b}

wobei das erfindungsgemäße Verfahren besonders effizient ist, wenn in den Elektrolytlösungen ein Gehalt an Alkalimetallcarbonaten und/oder -hydrogencarbonaten im Konzentrationsbereich von 0,02 bis 2 mol/l, bevorzugt 0,1 bis 1 mol/l, aufrechterhalten wird.

Gegenstand dieser Erfindung ist somit das Verfahren gemäß Anspruch 1.

Durch Variation der Zusammensetzung der Elektrolytlösung im Hinblick auf die Leitsalze Alkalimetallchlorid, Alkalimetallsulfat und Alkalimetallcarbonat bzw. -hydrogencarbonat kann die Elektrolyse bezüglich Elektrolysespannung und Reinheit des erzeugten basischen Kobalt(II)carbonats weitgehend optimiert werden. Unter optimalen Bedingungen kann die anodische Oxidation mit Stromdichten bis zu 2000 Am⁻² durchgeführt werden. Raum-Zeit-Ausbeuten bis zu 50 kg Co(II)/hm³ sind somit ohne weiteres erreichbar. Solche Raum-Zeit-Ausbeuten sind durch chemische Auflösung, insbesondere hochreinen Kobaltmetalls, nicht zu erreichen.

Bevorzugt enthalten die Elektrolytlösungen als Leitsalz Alkalimetallchloride im Konzentrationsbereich von 0,1 bis 5 mol/l, bevorzugt 0,2 bis 2 mol/l, und/oder Alkalisulfate im Konzentrationsbereich von 0 bis 0,1 mol/l und/oder Kobalt(II)chlorid bis maximal 0,1 mol/l.

Die Elektrolytlösungen weisen beim erfindungsgemäßen Verfahren bevorzugt Temperaturen im Bereich von 5 bis 80°C, bevorzugt 10 bis 30°C, auf. Bei niedrigeren Temperaturen sind Endprodukte mit geringerem Verunreinigungsgehalt erhältlich. Die ph-Werte der Elektrolytlösungen sollten im Bereich von 5 und 11, bevorzugt 6 und 9,5, gehalten werden.

Die Reinheit des elektrolytisch gewonnenen erfindungsgemäßen basischen Kobalt-(II)carbonats wird weiterhin durch die Verweilzeit in der Elektrolyse beeinflußt. Die in den Beispielen 1 bis 5 gewählte Verweilzeit von 1 h gewährleistet, daß die Verunreinigungen Natrium und Chlorid gut auswaschbar sind.

Bei der Beurteilung der aufzuwendenden Waschwassermengen muß berücksichtigt werden, daß mit dem basischen Kobalt(II)carbonat ungefähr 7 bis 10 I Elektrolytlösung pro kg Co in Form von anhaftender Feuchte aus der Elektrolyse entnommen werden. Diese Menge wird bei der Wäsche wieder aus dem Feststoff verdrängt, fließt in den Elektrolysekreislauf zurück und belastet die Abwasserbilanz nicht.

Die in der folgenden Aufarbeitung des Filterkuchens durchgeführte Heißanmaischung bewirkt eine weitergehende Senkung der Alkali- und Chloridwerte. Des weiteren wird bei der Erwärmung der basischen Kobalt(II)carbonate CO₂ freigesetzt, das aus wirtschaftlichen Gründen direkt in die Elektrolyse zurückgeführt werden kann. Das abgetrennte basische Kobalt(II)carbonat wird somit vorzugsweise bei Temperaturen zwischen 50 und 100°C angemaischt und erneut filtriert und gewaschen. Weiterhin können vorteilhaft bei der Anmaischung Alkalilaugen und/oder Ammoniak zugesetzt werden. Hierdurch kann eine weitere Absenkung des Chloridgehaltes erzielt werden. Außerdem wird hierdurch eine Substitution des Carbonatanions gegen Hydroxidanionen bewirkt. Bei mindestens stöchiometrischer Menge an Alkalilaugen bzw. Ammoniak kann reines Co(OH)₂ erhalten werden.

Die erfindungsgemäß erhältlichen basischen Kobalt(ll)carbonate der allgemeinen Formel

Co[(OH)₂]ₐ[CO₃]₁₋ₐ

weisen bevorzugt einen Gehalt an Leitsalzen von <800 ppm, besonders bevorzugt <200 ppm, auf.

Wird die Elektrolyse unter Zugabe definierter Mengen Oxalsäure oder Oxalate durchgeführt, können die entsprechenden oxalatdotierten basischen Kobalt(II)-carbonate der allgemeinen Formel

Co[(OH)₂]ₐ[C₂O₄]_{b}[CO₃]_{1-a-b}

erhalten werden. Bei Einsatz von Oxalsäure entstehen keinerlei zusätzliche Neutralsalze.

Im folgenden wird die Erfindung beispielhaft erläutert, ohne daß hierin ein Einschränkung zu sehen ist.

### Beispiele 1-5

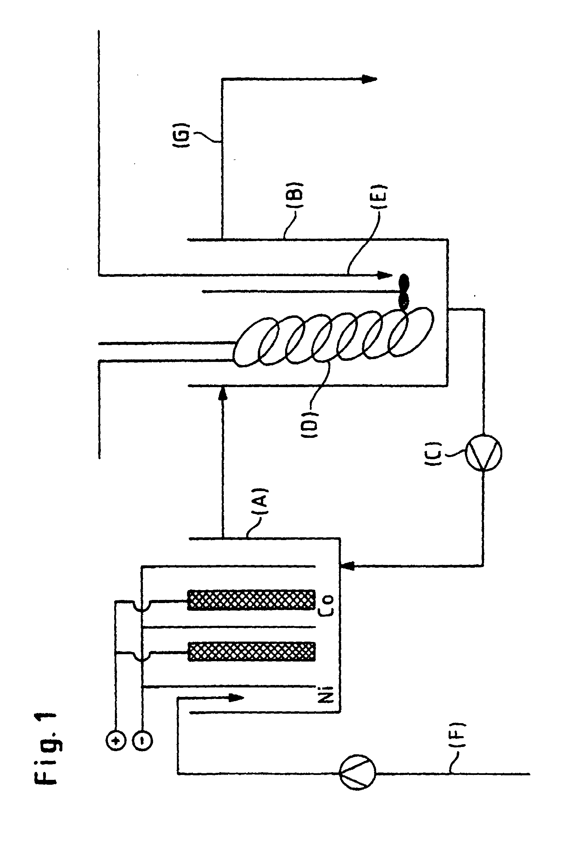

Die Versuche wurden in einer Elektrolyseapparatur, wie sie schematisch in Fig. 1 dargestellt ist, durchgeführt. Diese Elektrolyseapparatur bestand aus der eigentlichen Elektrolysezelle (A) und einem Umlaufbehälter (B). Über den Umlaufbehälter wurde die Elektrolyt/Produktsuspension mit einer Kreiselpumpe (C) von unten durch die Zelle (A) gepumpt, um eine gute Durchmischung zu erreichen.

Im Umlaufbehälter (B) war eine Kühlschlange (D) installiert, um entstehende Joule'sche Wärme abzuführen. Außerdem wurde Kohlendioxid durch eine Fritte (E) in die Elektrolysesuspension eingeleitet. Um vor Elektrolysebeginn die CO₂-Sättigung der Elektrolyselösung zu garantieren, wurde für eine Stunde CO₂ in die Elektrolyselösung eingeleitet, bevor der Elektrolysestrom eingeschaltet wurde. Der Zelle (A) wurde kontinuierlich frischer Elektrolyt (F) zugeführt. Über einen Überlauf (G) am Umlaufbehälter (B) floß die Produktsuspension kontinuierlich ab.

Die Elektrolysezelle (A) wurde mit zwei Anoden mit einer Gesamtfläche von 1200 cm² beschickt. Es kamen handelsübliche Kobaltelektroden zum Einsatz. Die verwendeten Kathoden der Elektrolysezelle bestanden aus 2 mm starken Reinstnickel- oder Kobaltblechen.

Proben wurden jeweils entnommen, nachdem die Feststoffkonzentration und Temperatur der Elektrolysesuspension einen stationären Zustand erreicht hatte.

Die Aufarbeitung erfolgte, indem die aus dem Umlaufbehälter (B) kontinuierlich ablaufende Produktsuspension auf einer Filternutsche vom Elektrolyten abfiltriert und der Filterkuchen zunächst mit kaltem destilliertem Wasser gewaschen wurde. Bei den Beispielen 1, 2 und 5 wurde der so gewonnene Filterkuchen einer weiteren Reinigung durch Anmaischen mit heißem destilliertem Wasser bzw. Natronlauge unterzogen. Die Suspension wurde heiß filtriert, der Filterkuchen mit Wasser gewaschen und bei 80°C im Trockenschrank bis zur Gewichtskonstanz getrocknet. Bei den Beispielen 3 und 4 wurde der so gewonnene Filterkuchen lediglich durch Waschen mit 80°C heißem destilliertem Wasser einer weiteren Reinigung unterzogen und abschließend bei 80°C im Trockenschrank bis zur Gewichtskonstanz getrocknet.

Die Elektrolytzusammensetzung, die Elektrolysebedingungen sowie die charakteristischen chemischen Analysen sind in der nachfolgenden Tabelle 1 zusammengefaßt.

Die in Tabelle 1 beschriebenen Elektrolysen wurden in einer Elektrolysezelle mit einem Bruttovolumen von 5,0 l durchgeführt. Bei einem Strom von 200 A (Beispiel 2, Tabelle 1) wurden 428,2 g basischen Kobalt(II)carbonats pro Stunde gebildet. Mit einem Kobaltgehalt von 51,1 Gew.-% entspricht dies 218,8 g Kobalt, entsprechend einer Stromausbeute von 99,6 %. Eine kathodische Kobaltabscheidung wurde nicht beobachtet Es trat auch keine anodische Chlorentwicklung auf. Unter diesen Elektrolysebedingungen ergab sich eine Raum-Zeit-Ausbeute von 43,8 kg Co(II)/h·m³.

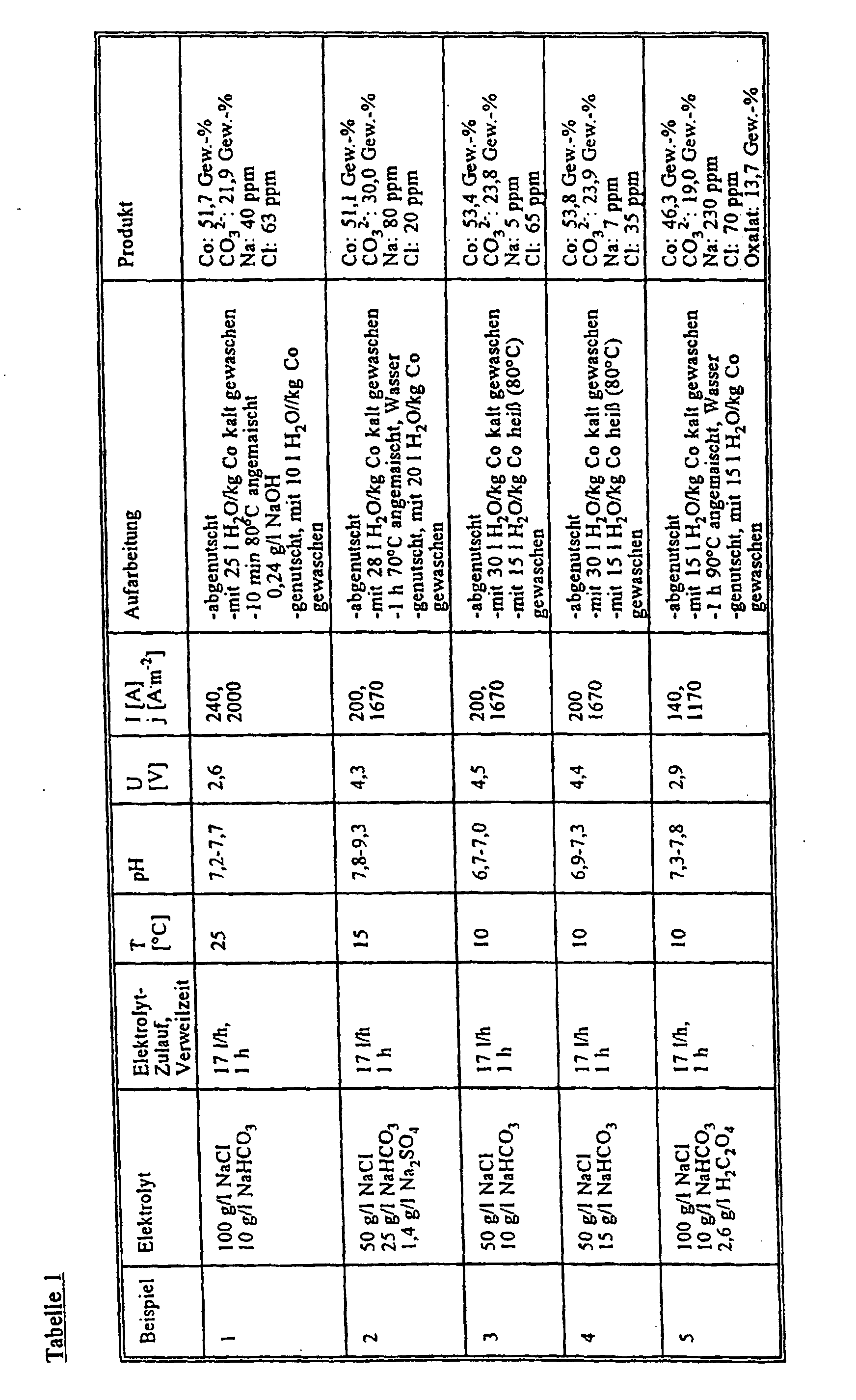

### Beispiel 6: Herstellung von Kobalt(II)hydroxid

500 g des feuchten Filterkuchens des basischen Kobalt(II)carbonats aus Beispiel 1 mit einem Co-Inhalt von 103 g wurden in 700 ml 10-Gew.-% iger Natronlauge suspendiert und unter Argon-Atmosphäre 1 h lang auf 80°C erhitzt. Die Suspension wurde heiß filtriert und der Filterkuchen mit 20 l Wasser pro kg Kobalt gewaschen. Nach der Trocknung des Filterkuchens bei 80°C im Vakuumtrockenschrank erhielt man 170,8 g rosafarbigen Pulvers. Die Röntgenbeugungsanalyse zeigte, daß es sich um phasenreines Co(II)hydroxid handelte. Der Co-Gehalt wurde zu 60,3 Gew.-% bestimmt, der Carbonatgehalt betrug 0,27 Gew.-%. Das Material wies einen Chloridgehalt von < 20 ppm und einen Natriumgehalt von 90 ppm auf.

### Beispiel 7: Herstellung von Kobaltmetall enthaltendem Kobalt(II)oxid

300 g basischen Kobalt(II)carbonats aus Beispiel 5 wurden in einem Quarzschiffchen unter Argon-Atmosphäre 2 h bei 620°C kalziniert. Man erhielt 171,4 g hellbraunen Pulvers. Die Röntgenbeugungsanalyse zeigte neben Kobalt(II)oxid einen geringen Anteil kubischen und hexagonalen Kobaltmetalls. Es konnte kein Co(III)-oxid nachgewiesen werden. Der Kobaltgehalt wurde zu 82,0 Gew.-% bestimmt.

### Beispiel 8: Herstellung von Kobalt(II)oxid

300 g basischen Kobalt(II)carbonats aus Beispiel 2 wurden in einem Quarzschiffchen 2 h unter Argon-Atmosphäre auf 650°C erhitzt. Man erhielt 195,1 g graubraunen Pulvers. Der Kobaltgehalt betrug 78,58 Gew.-%. In der Röntgenbeugunganalyse wurde nur Kobalt(II)oxid nachgewiesen.

### Beispiel 9: Herstellung von Kobaltmetallpulver

150 g basischen Kobalt(II)carbonats aus Beispiel 2 wurden in einem Quarzschiffchen unter Wasserstoff-Atmosphäre 3 h bei 650°C reduziert. Nach beendeter Reduktion ließ man unter Argon-Atmosphäre abkühlen. Man erhielt 77,0 g schwarzgrauen Pulvers. Der Kobaltgehalt wurde zu 99,6 Gew.-% ermittelt. Das Pulver hatte einen FSSS-Wert* von 2,8 µm.
*FSSS = Fisher Sub Sieve Siver ltd. ASTM B 330
(Messung des mittleren Korndurchmessers mittels Permeabilitätsmessung)

## Patentansprüche

1. Verfahren zur Herstellung basischer Kobalt(II)carbonate oder Kobalt(II)oxalatcarbonate der allgemeinen Formel
Co[(OH)₂]ₐ[C₂O₄]_{b}[CO₃]_{1-a-b}
durch anodische Oxidation von metallischem Kobalt in wäßrigen, CO₂-gesättigten Elektrolytlösungen, gegebenenfalls in Gegenwart von Oxalsäure und/oder Oxalaten, Abtrennung und Waschung des so erhaltenen basischen Kobalt(II)carbonats, **dadurch gekennzeichnet, daß** die Elektrolytlösungen Alkalimetallcarbonate und/oder -hydrogencarbonate im Konzentrationsbereich von 0,02 bis 2 mol/l, bevorzugt 0,1 bis 1 mol/l, enthalten.

2. Verfahren gemäß Anspruch 1, **dadurch gekennzeichnet, daß** die Elektrolytlösungen als Leitsalz Alkalimetallchloride im Konzentrationsbereich von 0,1 bis 5 mol/l, bevorzugt 0,2 bis 2 mol/l, enthalten.

3. Verfahren gemäß einem der Ansprüche 1 und 2, **dadurch gekennzeichnet, daß** die Elektrolytlösungen als Leitsalz Alkalimetallsulfate im Konzentrationsbereich von 0 bis 0,1 mol/l und/oder Kobalt(II)chlorid bis maximal 0,1 mol/l enthalten.

4. Verfahren gemäß einem der Ansprüche 1 bis 3, **dadurch gekennzeichnet, daß** die Elektrolytlösungen Temperaturen im Bereich von 5 bis 80°C, bevorzugt von 10 bis 30°C, aufweisen.

5. Verfahren gemäß einem der Ansprüche 1 bis 4, **dadurch gekennzeichnet, daß** in den Elektrolytlösungen ein pH-Wert zwischen 5 und 11, bevorzugt zwischen 6 und 9,5, aufrechterhalten wird.

6. Verfahren gemäß einem der Ansprüche 1 bis 5, **dadurch gekennzeichnet, daß** das abgetrennte basische Kobalt(II)carbonat bei Temperaturen zwischen 50 und 100°C angemaischt und erneut filtriert und gewaschen wird.

7. Verfahren gemäß Anspruch 6, **dadurch gekennzeichnet, daß** bei der Anmaischung Alkalilaugen und/oder Ammoniak zugesetzt werden.

## Claims

1. Process for producing basic cobalt(II) carbonates or cobalt(II) oxalate carbonates of the general formula
Co[(OH₂]ₐ[C₂O₄]_{b}[CO₃]₁-ₐ-_{b}
by anodic oxidation of metallic cobalt in aqueous CO₂-saturated electrolyte solutions, optionally in the presence of oxalic acid and/or oxalates, removal and washing of the basic cobalt(II) carbonate thus obtained, **characterized in that** the electrolyte solutions contain alkali metal carbonates and/or bicarbonates in the concentration range from 0.02 to 2 mol/l, preferably 0.1 to 1 mol/l.

2. Process according to Claim 1, **characterized in that** the electrolyte solutions contain, as conducting salt, alkali metal chlorides in a concentration range of 0.1 to 5 mol/l, preferably 0.2 to 2 mol/l.

3. Process according to either of Claims 1 and 2, **characterized in that** the electrolyte solutions contain, as conducting salt, alkali metal sulphates in a concentration range of 0 to 0.1 mol/l and/or cobalt(II) chloride up to a maximum of 0.1 mol/l.

4. Process according to any of Claims 1 to 3, **characterized in that** the electrolyte solutions have temperatures in the range of 5 to 80°C, preferably 10 to 30°C.

5. Process according to any of Claims 1 to 4, **characterized in that** a pH of between 5 and 11, preferably between 6 and 9.5, is maintained in the electrolyte solutions.

6. Process according to any of Claims 1 to 5, **characterized in that** the removed basic cobalt(II) carbonate is slurried up at temperatures between 50 and 100°C, is again filtered and washed.

7. Process according to Claim 6, **characterized in that** alkali liquors and/or ammonia are added during mashing.

## Revendications

1. Procédé de fabrication de carbonates basiques de cobalt (II) ou d'oxalates-carbonates de cobalt (II) de formule générale
CO[(OH)₂]ₐ[C₂O₄]_{b}[CO₃]_{1-a-b}
par oxydation anodique de cobalt métallique dans des solutions aqueuses d'électrolyte, saturées en CO₂, le cas échéant en présence d'acide oxalique et/ou d'oxalates, séparation et lavage du carbonate basique de cobalt (II) ainsi obtenu, **caractérisé en ce que** les solutions d'électrolyte contiennent des carbonates et/ou des hydrogénocarbonates de métaux alcalins dans une plage de concentration de 0,02 à 2 moles/l, de préférence de 0,1 à 1 mole/l.

2. Procédé suivant la revendication 1, **caractérisé en ce que** les solutions d'électrolyte contiennent comme sel conducteur des chlorures de métaux alcalins dans une plage de concentration de 0,1 à 5 moles/l, de préférence de 0,2 à 2 moles/l.

3. Procédé suivant l'une des revendications 1 ou 2, **caractérisé en ce que** les solutions d'électrolyte contiennent comme sel conducteur des sulfates de métaux alcalins dans une plage de concentration de 0 à 0,1 mole/l et/ou du chlorure de cobalt (II) jusqu'à maximum 0,1 mole/l.

4. Procédé suivant l'une des revendications 1 à 3, **caractérisé en ce que** les solutions d'électrolyte présentent des températures dans une plage de 5 à 80°C, de préférence de 10 à 30°C.

5. Procédé suivant l'une des revendications 1 à 4, **caractérisé en ce qu'**on maintient dans les solutions d'électrolyte un pH entre 5 et 11, de préférence entre 6 et 9,5.

6. Procédé suivant l'une des revendications 1 à 5, **caractérisé en ce qu'**on trempe le carbonate basique de cobalt (II) séparé à des températures entre 50 et 100°C et qu'on le filtre et le lave à nouveau.

7. Procédé suivant la revendication 6, **caractérisé en ce qu'**on ajoute des lessives alcalines et/ou de l'ammoniaque lors du trempage.
